(19) Europäisches Patentamt
European Patent Office
Office européen des brevets

(11) Publication number: 0 128 736
B1

(12) # EUROPEAN PATENT SPECIFICATION

(45) Date of publication of the patent specification: 25.02.87

(51) Int. Cl.⁴: C 07 D 277/18, C 07 D 277/48 // A61K31/425

(21) Application number: 84303793.8

(22) Date of filling: 05.06.84

(54) **Novel 2-guanidinothiazoline compounds, their preparation, and their use as intermediates.**

(30) Priority: 07.06.83 JP 102206/83

(43) Date of publication of application: 19.12.84 Bulletin 84/51

(45) Publication of the grant of the patent: 25.02.87 Bulletin 87/9

(84) Designated Contracting States: FR GB IT

(56) References cited:
CHEMICAL ABSTRACTS, vol. 94, no. 17, April 27, 1981, Columbus, Ohio, USA, HIRATA, YESUTUMI, "Guanitinothiazolo compounds", page 765, column 1, abstract no. 139 794 w
CHEMICAL ABSTRACTS, vol. 90, no. 11, March 12, 1979, Columbus, Ohio, USA, YELLIN TOBIAS "Guanidine derivatives", page 626, column 1,2, abstract-no. 87 452d

(73) Proprietor: YAMANOUCHI PHARMACEUTICAL CO. LTD., No. 5-1 Nihonbashi- Honcho, 2-chome Chuo- ku, Tokyo (JP)

(72) Inventor: Hirata, Yasufumi, No.15- 107, 477- 17, Konba- cho, Omiya- shi Saitama (JP)
Inventor: Yanagisawa, Isao, No.22- 8, Shakujiidai 2-chome Nerima- ku, Tokyo (JP)

(74) Representative: Geering, Keith Edwin, REDDIE & GROSE 16 Theobalds Road, London WC1X 8PL (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convension).



LIBER, STOCKHOLM 1987

**Description**

The present invention relates to novel 2-guanidinothiazoline compounds and acid addition salts thereof.

According to this invention, there are provided 2-guanidinothiazoline compounds of the general formula

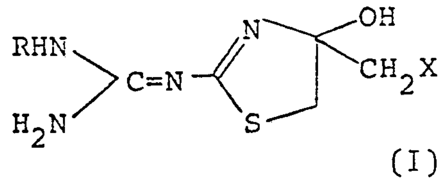

(I)

Wherein R represents a hydrogen atom or a lower alkyl group, and X represents a halogen atom.

The term "lower" in the above definition means a straight or branched carbon chain having 1-5 carbon atoms. Suitable lower alkyl groups include a methyl group, an ethyl group, an isopropyl group, a butyl group, etc. The halogen atoms may e.g. be chlorine, bromine or iodine.

Furthermore, the compounds of the general formula I can form acid addition salts and there also exist the tautomers thereof. The invention includes such acid addition salts and tautomers.

The acid addition salts include the salts of the compounds with inorganic acids such as hydrochloric acid, hydrobromic acid, sufuric acid, etc., and with aliphatic carboxylic acids, for example, acetic acid, maleic acid, fumaric acid, etc.

The compounds of the general formula I and the acid addition salts thereof provided by this invention are important intermediate compounds useful for preparing famotidine (cf. unexamined Japanese patent applications laid open under Nos. 56-22770 and 56-55383) and thiotidine (cf. unexamined Japanese patent application laid open under No. 53-147069) which are useful compounds for medical purposes as histamine H-2 receptor blockers or gastric acid secretion inhibitors.

Hitherto, as an intermediate compound for preparing such compounds, 2-guanidino-4-chloromethylthiazole (hereinafter referred to as "Compound A") is known from unexamined Japanese patent application laid-open under No. 53-147069. However, Compound A is undesirable in that handling of the same is complicated since it has an irritative odor and causes contact dermatitis.

When the compounds of this invention shown by general formula I are used as intermediate compounds for preparing famotidine or thiotidine, the above problem connected with Compound A (that is, the problem of complicated handling) is avoided. In addition, the compounds of general formula I have the advantage that they can be obtained in high yield and are easy to purify.

The compounds of this invention can be produced by the following process.

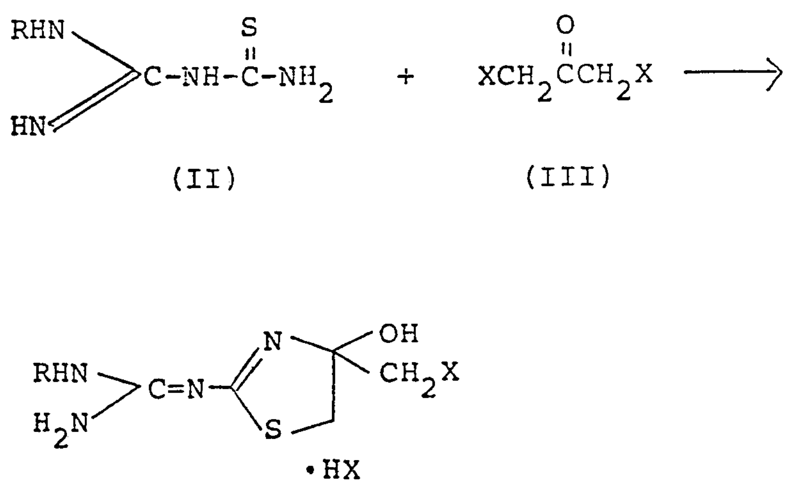

(II) (III)

(I)

This process may involve reacting starting material of formula II and a reactive amount of 1,3-dihalogenoacetone of formula III in an organic solvent under cooling. Any anhydrous organic solvents which do

not take part in the reaction may be used but acetone is preferably used. It is preferred that the reaction temperature be maintained between 0°C and -10°C. In order to obtain the desired compound, the solid material formed in the reaction mixture is collected by filtration, and washed with solvent e.g. organic solvent such as acetone. The material thus obtained is pure enough to use for the next process as the intermediate material compound.

The following Examples will serve to illustrate the present invention, and the following Reference Examples will further serve to illustrate the preparation of famotidine using the compounds of formula I. In the Examples and Reference Examples, m.p., Anal. and NMR. are abbreviations for melting point, elementary analysis values and nuclear magnetic resonance spectrum, respectively.

Example 1

N"-(4-(chloromethyl)-4,5-dihydro-4-hydroxy-2- thiazolyl)-guanidine hydrochloride

60.0 kg of dichloroacetone is dissolved in 550 1 of acetone. After cooling the solution to -5 ~ -7°C, 55.8 kg of (aminoiminomethyl)thiourea [amidinothiourea] is added to the solution under cooling in 10 kg amounts at hourly intervals.

The mixture is stirred continuously for 5 days below 0°C. The resultant precipitates are collected by filtration, and washed with 50 l of acetone to provide 111.6 kg of the desired compound. This material can be used as the starting material for the next process.

IR (KBr), $\nu_{max}$ 3200, 2850, 1680, 1595 $cm^{-1}$ NMR (DMSO-$d_6$) $\delta$:

3.52 ($AB_q$, J = 12HZ, 2H, -S-$C\underline{H}_2$-)

3.80 (S, 2H, -$C\underline{H}_2$C1)

6.96 (bs, 1H, -O$\underline{H}$)

8.04 (bs, 4H, 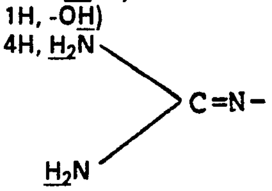

9.60 (bs, 1H, $\underline{H}$C1)

Reference Example 1

N" [4-([(aminoiminomethyl)thio)methyl]-2-thiazolyl]-guanidine dihydrochloride

In 500 ml of water are dissolved 111.6 kg of the material obtained in Example 1 and 32.9 kg of thiourea. The solution is stirred for one hour at 50° ~ 55°C to complete the reaction. (N'-[4-[[(aminoiminomethyl)thio]methyl]-2-thiazolyl]-guanidine dihydrochloride is formed in the reaction mixture, and this reaction mixture is directly used for the next process without isolation of the formed compound.

Reference Example 2

N"-[4-[((2-cyanoethyl)thio]methyl]-2-thiazolyl]- guanidine

The reaction mixture obtained in Reference Example 1 is cooled below 10°C, and to the solution are added 45.6 kg of β-chloropropionitrile and 200 l of isopropanol. A solution of 69,1 kg of sodium hydroxide in 280 l of water is added dropwise to the solution under nitrogen stream followed by stirring for 2 hours at 0°C ~ 10°C. The crystals precipitated are collected by filtration, and washed with cold water and dried to provide 91.7 kg of the desired compound. m.p. 125 - 126.5°C.

Reference Example 3

Methyl 3-[[[2-[(diaminomethylene)amino]-4-thiazolyl]- methyl]thio]propionimidate

In 60 l of anhydrous dimethylformamide is dissolved 34.3 kg of the material formed in Reference Example 2. After adding 60 l of anhydrous methanol to the solution, 61.9 kg of hydrogen chloride gas is passed through the solution below 5°C. After stirring the reaction mixture for 2 days at 0°C. ~ 5°C, the reaction mixture is poured into a mixture of 350 l of water, 250 kg of potassium carbonate, 30 l of ethyl acetate and ice while stirring below 5°C. The reaction mixture is stirred for 2 hours at 0 ~ 5°C, and the resultant precipitates are collected by filtration. After stirring a mixture of the precipitates and 400 1 of water for 0.5 hour at 0°C ~ 5°C, the resultant precipitates are collected by filtration, washed with 40 l of water and 10 l of cooled acetone respectively, and dried at reduced pressure to provide 30.6 kg of the desired product showing a melting point of 125.7°C.

Reference Example 4

3-[([2-(diaminomethylene)amino]-4-thiazolyl]methyl]thio]-N-sulfamoylpropionamidine (generic name: famotidine)

In 340 l of methanol is dissolved 88.4 kg of sulfamide under heating, and the solution is cooled to 30°C. To the solution, 114.2 kg of the material obtained in Reference Example 3 are added in three portions while stirring at 20 ~ 30°C; the second portion is added 8 hours after the first, and the third portion 24 hours after the first.

After stirring the reaction mixture for a further 2 days at 20° ~ 30°C, the crystals formed are collected by filtration, washed with 200 l of cooled methanol, and air-dried at room temperature to provide 87.5 kg of the desired product showing a melting point of 157.6°C. Some of the obtained product is recrystallized from dimethylformamide-water, and is dissolved in an equivalent molar amount of aqueous acetic acid. To the solution is added an equivalent molar amount of a dilute sodium hydroxide solution in water to separate crystals showing the following physicochemical properties:

I) m.p. 163 ~ 164°C

II) Anal. (for $C_8H_{15}N_7O_2S_3$)

| | C(%) | H(%) | N(%) |
|---|---|---|---|
| Calculated: | 28.48 | 4.48 | 29.06 |
| Found: | 28.37 | 4.48 | 28.97 |

III) NMR (DMSO-$d_6$)
2.50 (2H, m, $-SCH_2\underline{H_2}-$)
2.65 (2H, m, $-S\underline{CH_2}CH_2-$)

3.60 (2H, s, $\underline{CH_2}S-$ 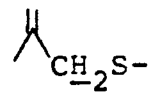 )

6.45 (1H, s, 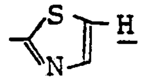 )

IV) Mass. (FD method) m/e 338

Reference Examples 5 and 6 below produce the same products as Reference Examples 1 and 2 above using different reaction conditions:

Reference Example 5

N''-(4-[[(aminoiminomethyl)thio]methyl]-2-thiazolyl]-guanidine dihidrochloride

To 111.6 kg of the material obtained in Example are added 500 l of ethyl alcohol and further 31.0 kg of thiourea. The solution is stirred for one hour at 50 ~ 55°C, further refluxed under heating for 0.5 hour, and then cooled to 5°C. The crystals formed are collected by filtration and washed with 60 l of cold ethyl alcohol and air-dried at room temperature to provide 103.5 kg of the desired product showing a melting point of 201 ~ 207°C.

Reference Example 6

N''[4-[[(2-cyanoethyl)thio]methyl]-2-thiazolyl]-guanidine

In a mixture of 170 l of iso-propyl alcohol and 500 l of water are dissolved 67.1 kg of the material obtained in Reference Example 5. To the solution are added 23.8 kg, of β -chloropropionitrile followed by adding a solution of 35.4 kg of sodium hydroxide in 160 l of water under nitrogen gas atmosphere below 10°C. The reaction mixture is stirred for 1 hour below 10°C, then for 2 hours at 10-15°C, and then cooled to 5°C. The precipitate formed is collected by filtration, and washed with 130 l of water and 30 l of cold iso-propyl alcohol respectively, and then aair-dried at room temperature to provide 42.3 kg of the desired product showing a melting point of 125.5°C.

Thus in addition to the formula I compounds and their acid addition salts, the invention provides a method for their preparation. It further provides their use as intermediates for the preparation of corresponding famotidine and thiotidine compounds (including famotidine and thiotidine themselves when R is hydrogen); one famotidine compound preparation according to the invention comprises (a) reacting a compound of formula I or an acid addition salt thereof with thiourea (R in formula I being hydrogen for the production of famotidine itself), (b) reacting the reaction (a) product with β-chloropropionitrile in the presence of isopropanol, (c) reacting the reaction (b) product with hydrogen chloride, and (d) reacting the reaction (c) product with sulfamide; a preferred method for the preparation of famotidine comprises (a) reacting the compound of the formula

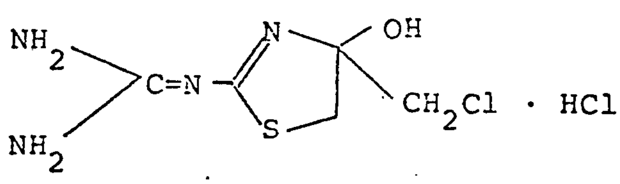

with thiourea to obtain the compound N''-[4-[[(aminoiminomethyl)thio]methyl]-2-thiazolyl]-guanidine dihydrochloride;

(b) reacting the compound produced in step (a) with β-chloropropionitrile in the presence of isopropanol to obtain the compound N''-[4-[[(2-cyanoethyl)thio]methyl]-2-thiazolyl]-guanidine;

(c) reacting the compound produced in step (b) with hydrogen chloride to obtain the compound methyl 3-[[[2-[(diaminomethylene)amino];4-thiazolyl]-methyl]thio]propionimidate; and (d) reacting the compound produced in step (c) with sulfamide to obtain famotidine.

**Claims:**

1. 2-Guanidinothiazoline compounds of the general formula

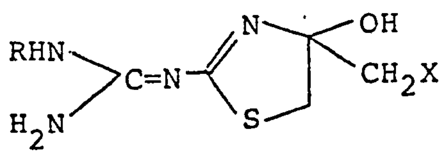

wherein R represents a hydrogen atom or a $C_1$-$C_5$ alkyl group and X represents a halogen atom, and the acid addition salts thereof.

2. N''-[4-(chloromethyl)-4,5-dihydro-4-hydroxy-2-thiazolyl]-guanidine according to claim 1.

3. A process for preparing a 2-guanidinothiazoline compound of the formula

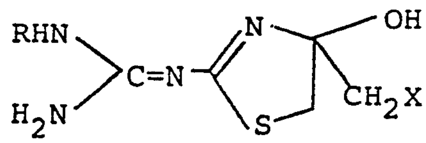

as defined in claim 1 or an acid addition salt thereof which comprises reacting 1,3-dihalogenacetone of the general formula

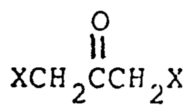

wherein X represents a hologen atom, and amidinothiourea of the general formula

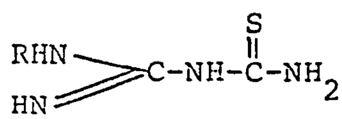

wherein R represents a hydrogen atom or a $C_1$ to $C_5$ alkyl group.

4. A process for preparing famotidine comprising:

(a) reacting the compound of the formula

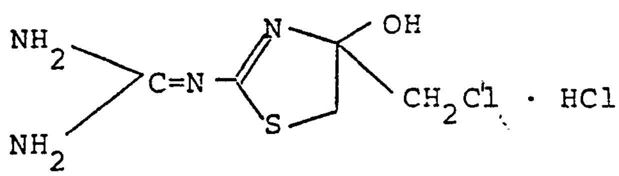

with thiourea to obtain the compound N''[4-[[(aminoiminomethyl)thio]methyl] -2-thiazolyl]-guanidine dihydrochloride;

(b) reacting the compound produced in step (a) with β-chloropropionitrile in the presence of isopropanol to obtain the compound N''[4-[[(2-cyanoethyl)thio]methyl]-2-thiazolyl] guanidine;

(c) reacting the compound produced in step (b) with hydrogen chloride to obtain the compound methyl 3-[[[2-[(diaminomethylene)amino]-4-thiazolyl]-methyl]thio]propionimidate; and

(d) reacting the compound produced in step (c) with sulfamide to obtain famotidine.

5. A process for preparing a famotidine compound which comprises (a) reacting a compound according to claim 1 with thiourea, (b) reacting the reaction (a) product with β-chloropropionitrile in the presence of

isopropanol, (c) reacting the reaction (b) product with hydrogen chloride, and (d) reacting the reaction (c) product with sulfamide.

**Patentansprüche**

2-Guanidinothiazolin-Verbindungen der allgemeinen Formel

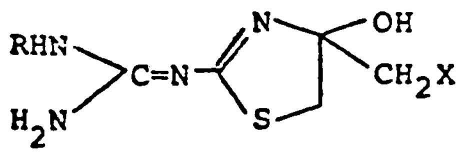

in der R ein Wasserstoffatom oder eine $C_1$-$C_5$ Alkylgruppe und X ein Halogenatom bedeutet, und deren Säureadditionssalze.

2. N''-(4-Chlormethyl)-4,5-dihydro-4-hydroxy-2-thiazolyl)-guanidin nach Anspruch 1.

3. Verfahren zur Herstellung einer 2-Guanidinothiazolin-Ver-bindung der Formel

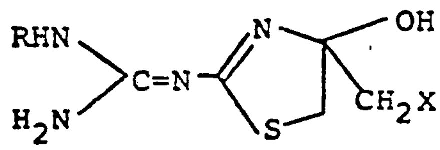

wie in Anspruch 1 definiert oder eines Säuresalzes davon, umfassend die Umsetzung von 1,3-Dihalogenaceton der allgemeinen Formel

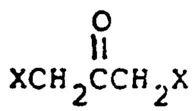

in der X ein Halogenatom bedeutet, mit Amidinothio-Harnstoff der allgemeinen Formel

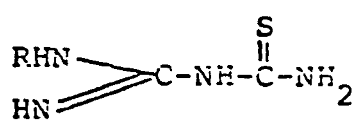

in der R ein Wasserstoffatom oder eine $C_1$ bis $C_5$ Alkylgruppe bedeutet.

4. Verfahren zur Herstellung von Famotidin umfassend a) die Umsetzung der Verbindung der Formel

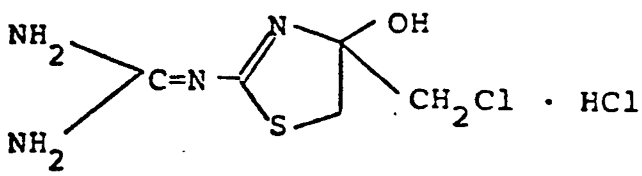

mit Thio-Harnstoff unter Bildung der Verbindung N''-[4-[[(Aminoiminomethyl)-thio]methyl]-2-thiazolyl]guanidin - dihydrochlorid;

b) Umsetzung der in Stufe (a) erhaltenen Verbindung mit β-Chloropropionitril in Gegenwart von Isopropanol unter Bildung der Verbindung N''-[4-[[(2-Cyanoethyl)thio]methyl]-2-thiazolyl]-guanidin,

c) Umsetzung der in Stufe (b) erhaltenen Verbindung mit Chlorwasserstoff unter Bildung der Verbindung Methyl -3-[[[2-(diamino - ethylen)amino]-4-thiazolyl)-methyl]thio]propionimidat) und d) Umsetzung der in

Stufe (c) erhaltenen Verbindung mit Sulfamid unter Bildung von Famotidin.

5. Verfahren zur Herstellung einer Famotidin-Verbindung, umfassend a) die Umsetzung einer Verbindung nach Anspruch 1 mit Thio-Harnstoff, b) Umsetzung des Reaktionsproduktes (a) mit β-Chlorpropionitril in Gegenwart von Isopropanol, C) Umsetzung des Reaktionsproduktes (b) mit Chlorwasserstoff und d) Umsetzung des Reaktionsproduktes (c) mit Sulfamid.

**Revendications**

1. Les composés de 2-guanidinothiazoline de formule générale:

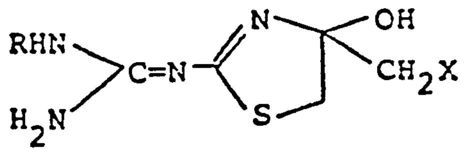

dans laquelle R représente un atome d'hydrogène ou un groupe alkoyle en $C_1$ à $C_5$ et X représente un atome d'halogène, et leurs sels d'addition acide.

2. La N''-[4-(chlorométyl)-4,5-dihydro-4-hydroxy-2-thiazolyl]-guanidine selon la revendication 1.

3. Un procédé de préparation d'un composé de 2-guanidinothiazoline de formule:

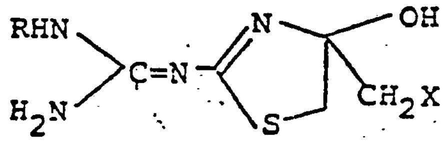

comme défini dans la revendication 1 ou leur sel d'addition acide qui consiste à faire réagir la 1,3-dihalogénoacétone de formule générale:

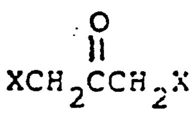

dans laquelle X représente un atome d'halogène, et l'amidinothiourée de formule générale:

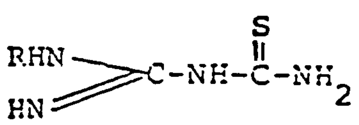

dans laquelle R représente un atome d'hydrogène ou un groupe alkoyle en $C_1$ à $C_5$.

4. Un procédé pour préparer la famotidine consistant à:

(a) faire réagir le composé de formule:

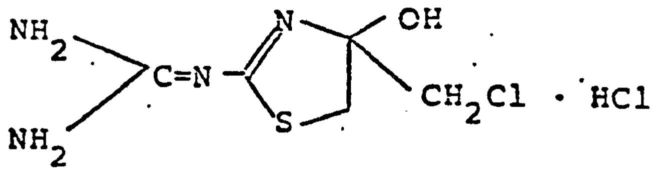

avec la thio-urée pour obtenir le dichlorhydrate de N''-[4-[[(aminoiminométhyl)thio]méthyl]-2-thiazolyl]-guanidine;

(b) faire réagir le composé produit au stade (a) avec du β-chloropropionitrile en présence d'isopropanol pour obtenir le composé N''-[4-[[(2-cyanoéthyl)thio]métyl]-2-hiazolyl]-guanidine;

(c) faire réagir le composé produit au stade (b) avec du chlorure d'hydrogène pour obtenir le 3-[[[2-[(diaminométhylène) amino]-4-thiazolyl]-méthyl]thio]propionimidate de méthyl; et

(d) faire réagir le composé produit au stade (c) avec du sulfamide pour obtenir la famotidine.

5. Un procédé de préparation d'un composé de famotidine qui consiste à (a) faire réagir un composé selon la revendication 1 avec de la thio-urée, (b) faire réagir le produit de la réaction (a) avec du β-chloropropionitrile en présence d'isopropanol, (c) faire réagir le produit de la réaction (b) avec du chlorure d'hydrogène, et (d) faire réagir le produit de la réaction (c) avec du sulfamide.